# EUROPEAN PATENT SPECIFICATION

(11) **EP 3 923 819 B1**
(45) Date of publication and mention of the grant of the patent: **12.08.2026**
(21) Application number: 20730857.8
(22) Date of filing: 15.05.2020
(51) Int. Cl.: A61B 8/12

(54) **APPARATUS TO PROVIDE AN ADJUSTABLE MECHANISM FOR RADIAL ULTRASOUND PORT AND FLUSH PORT**
VORRICHTUNG ZUR BEREITSTELLUNG EINES EINSTELLBAREN MECHANISMUS FÜR EINEN RADIALEN ULTRASCHALPORT UND SPÜLPORT
APPAREIL POUR FOURNIR UN MÉCANISME RÉGLABLE POUR ORIFICE ULTRASONORE RADIAL ET ORIFICE DE RINÇAGE

(30) Priority: 17.05.2019 US 201962849311 P; 17.05.2019 US 201962849649 P; 17.05.2019 US 201962849307 P
(43) Date of publication of application: 22.12.2021
(73) Proprietor: Boston Scientific Scimed, Inc., Maple Grove, MN 55311 (US)
(72) Inventor: WALSH, Kevin, Wellesley, Massachusetts 02482 (US); GARRITY, Douglas W., Waltham, Massachusetts 02453 (US)
(74) Representative: Vossius & Partner Patentanwälte Rechtsanwälte mbB
(86) International application number: PCT/US2020/033147
(87) International publication number: WO 2020/236595

(56) References cited:
- US-A- 4 723 550
- US-A1- 2005 038 410
- US-A1- 2016 331 468
- US-A1- 2016 361 528
- US-A1- 2017 007 203
- US-A1- 2018 279 868
- US-A1- 2018 279 994

## Description

### RELATED APPLICATIONS

### FIELD

The present disclosure relates generally to the field of medical devices. In particular, the present disclosure relates to an assembly with an adjustable ultrasound port and flush port for lateral and axial positioning of a radial ultrasound probe within a patient.

### BACKGROUND

A port for a radial ultrasound catheter may lack the ability to maintain visual imaging of pulmonary nodules within the peripheral regions of the lung while simultaneously repositioning a port for a radial ultrasound probe and/or port for flushing fluid through a lumen or lumens of the radial ultrasound catheter housing the same.

US 2018/0279994 A1discloses handle assemblies that allow a steerable shaft to be steered, while allowing separate movement of a medical tool. The handle assemblies can allow for rotation and axial movement of the medical tool. An actuator may be disposed distal to a second actuator that controls steering of the steerable shaft.

US 4 723 550 A discloses a hemostasis valve in which the valve member is a tubular, resilient gasket which may be compressed with variable pressure to provide a variable pressure seal to an elongated member extending through the tubular bore of the gasket. The gasket bore also defines at least one resilient, annular rib whereby the elongated member passing through the tubular gasket bore may be subjected at all times to at least a relatively low pressure seal from the resilient, annular rib, irrespective of the pressure of the variable pressure seal. Additionally, the pressure on the gasket may be applied and released by means of a pressure member positioned against the gasket. Structure is provided permitting longitudinal motion, but preventing rotational motion, of the pressure member. A rotatable cap is provided, with facing, mating spiral track means carried on both the inner face of the rotatable cap and outer face of the pressure member, to cause pressurizing advancement of the pressure member against the tubular gasket by rotation of the cap in one direction, and release of such pressurizing advancement by rotation of the cap in the other direction.

A variety of advantageous medical outcomes may therefore be realized by the adjustable assemblies, systems and methods of use thereof, of the present disclosure.

### SUMMARY

The invention is defined by the features of the independent claims.

In one aspect, the present disclosure relates to an adjustable probe assembly comprising a housing defining an internal chamber. An ultrasound port may be formed within a proximal portion of the housing. The ultrasound port may be coextensive with the internal chamber. A flush port may be disposed along a middle portion of the housing. The flush port may define a fluid channel therethrough. The fluid channel may be coextensive with the internal chamber. A fitting may be disposed around a distal portion of the housing. The housing may be configured to rotate relative to the fitting to alter an axial position of the flush port relative to the fitting.

In the described and other embodiments, an outer surface of the distal portion of the housing may include a surface feature configured to frictionally engage an inner surface of the fitting. The fitting may include a projection. A first seal may be disposed within a distal portion of the internal chamber. A second seal may be disposed within a proximal portion of the internal chamber. A bearing may be disposed within a proximal portion of the internal chamber and proximal to the second seal. The housing and ultrasound port may be configured to receive a radial ultrasound probe therethrough. The bearing may be configured to support rotation of the radial ultrasound probe. The radial ultrasound probe may include a drive cable. The drive cable may extend through the ultrasound port and may be supported by the bearing. The radial ultrasound probe may include a sheath. A proximal end of the sheath may be disposed within the housing between the first and second seals. The housing may be configured to rotate relative to the fitting to alter an axial position of the radial ultrasound probe. The proximal portion of the housing may be configured to rotate relative to a remaining portion of the housing to alter an axial position of the radial ultrasound probe.

In another aspect, the present disclosure relates to a system comprising a handle of a catheter. An adjustable probe assembly may be attached to the handle. The adjustable probe assembly may be configured to move laterally and axially relative to the handle. A radial ultrasound probe may extend through the adjustable probe assembly, the handle and a shaft of the catheter.

In the described and other embodiments, the adjustable probe assembly may include a housing defining an internal chamber. An ultrasound port may be formed within a proximal portion of the housing. A flush port may be disposed along a middle portion of the housing. The radial ultrasound probe may include a drive cable. The drive cable may extend through the ultrasound port. The flush port may include a fluid channel configured to deliver fluid into the internal chamber and through a sheath of the radial ultrasound probe. A first seal may be disposed within a distal portion of the internal chamber. A second seal may be disposed within a proximal portion of the internal chamber. The first and second seals may prevent fluid introduced through the flush port from exiting the internal chamber.

In yet another aspect, the present disclosure relates to a method comprising attaching an adjustable probe assembly to a handle of a catheter such that a radial ultrasound probe attached to the adjustable probe assembly may extend through the handle and a shaft of the catheter. A lateral position of the adjustable probe assembly may be adjusted relative to the handle. An axial position of the adjustable probe assembly may be adjusted relative to the handle.

In the described and other embodiments, a lateral position of the adjustable probe assembly may be re-adjusted relative to the handle to alter a lateral position of the radial ultrasound probe within a lumen of the catheter shaft. A housing of the adjustable probe assembly may be rotated relative to the handle to rotate the radial ultrasound probe within the lumen of the catheter. A housing of the adjustable probe assembly may be rotated relative to the handle to alter an axial position of a flush port disposed along a middle portion of the housing relative to the handle. A fluid may be introduced through the flush port and into an internal chamber of the adjustable probe assembly.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIGS. 1A-1B provide perspective (FIG. 1A) and cross-sectional (FIG. 1B) views of an adjustable probe assembly, according to one embodiment of the present disclosure.
FIGS. 2A-2B provide perspective (FIG. 2A) and cross-sectional (FIG. 2B) views of a radial ultrasound probe disposed within an adjustable probe assembly, according to one embodiment of the present disclosure.
FIGS. 3A-3D provide cross-sectional (FIG. 3A) and perspective (FIGS. 3B-3D views of an adjustable probe assembly attached to a handle of a catheter with radial ultrasound and needle biopsy capability, according to one embodiment of the present disclosure.

### DETAILED DESCRIPTION

The present disclosure is not limited to the particular embodiments described herein. The terminology used herein is for the purpose of describing particular embodiments only, and is not intended to be limiting beyond the scope of the appended claims. Unless otherwise defined, all technical terms used herein have the same meaning as commonly understood by one of ordinary skill in the art to which the disclosure belongs.

Although embodiments of the present disclosure are described with specific reference to probe assemblies, systems and methods of use thereof, and particularly leak-proof assemblies, which include an adjustable ultrasound port and/or flush port designed to maintain visual imaging of a peripheral pulmonary nodule while allowing lateral and/or axial repositioning of the radial ultrasound probe and/or flush port, it should be appreciated that such probe assemblies, systems and methods may be used to visualize and manipulate a variety of tissues within a variety of different body lumens and/or body passages.

As used herein, the singular forms "a," "an," and "the" are intended to include the plural forms as well, unless the context clearly indicates otherwise. It will be further understood that the terms "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof.

As used herein, the term "distal" refers to the end farthest away from the medical professional or physician when introducing a device into a patient, while the term "proximal" refers to the end closest to the medical professional or physician when introducing a device into a patient.

In various embodiments, the present disclosure relates generally to an adjustable and leak-proof probe assembly configured for use with a bronchial radial ultrasound system to provide real-time imaging and targeting of difficult to access pulmonary nodules. For example, the adjustable probe assembly may include an adjustable ultrasound port and flush port configured to allow a physician to laterally (*e.g.,* along a longitudinal axis) and/or axially (*e.g.,* about or around a longitudinal axis) position/reposition components of the bronchial radial ultrasound system (*e.g.,* ultrasound probe, flush port and/or probe assembly) within a peripheral region of the lung while maintaining a leak-proof seal to simultaneously flush fluid through a lumen of a radial ultrasound probe.

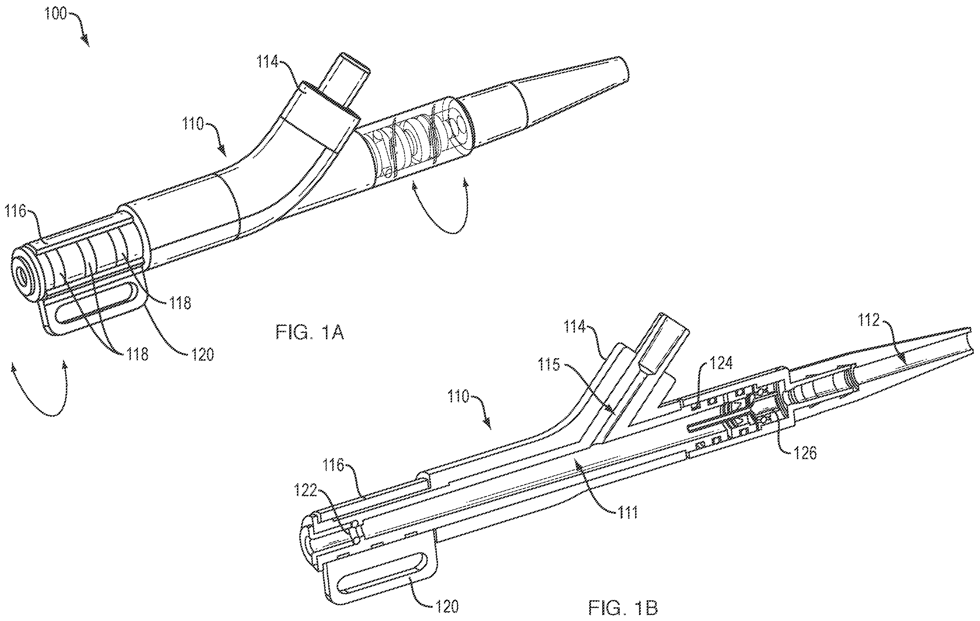

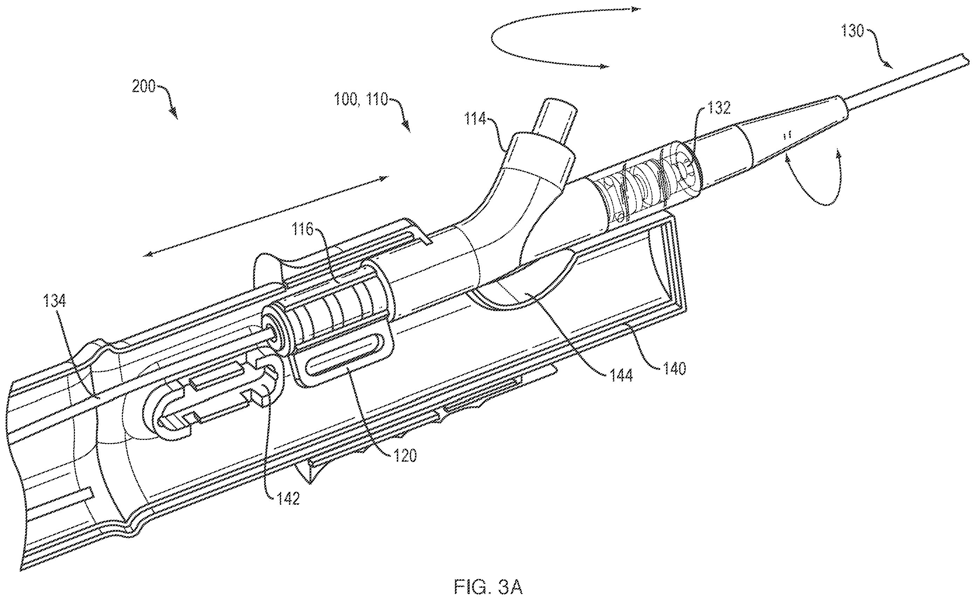

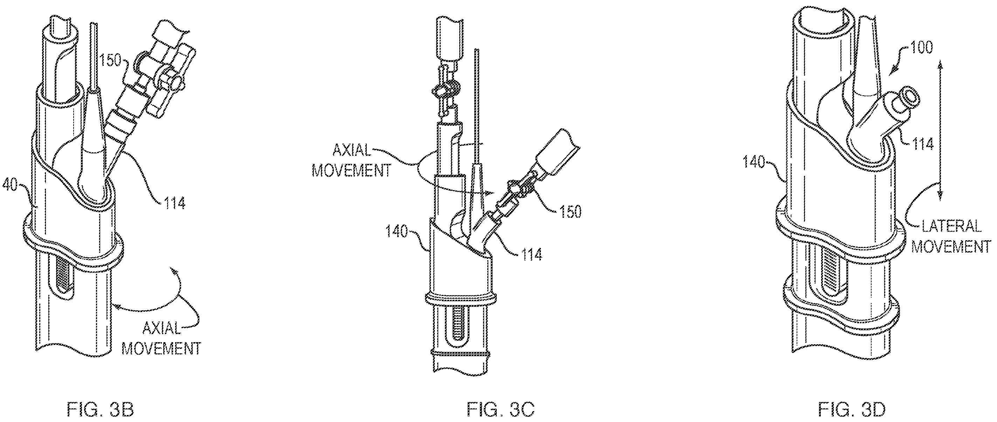

Referring to FIGS. 1A-1B, in one embodiment, an adjustable probe assembly 100 of the present disclosure may include a housing 110 defining an internal chamber 111. An ultrasound port 112 (*e.g.,* first port) may be formed within or otherwise extend through a proximal portion of the housing 110. In various embodiments, the ultrasound port 112 may be coextensive (*e.g*., substantially aligned with, etc.) with the internal chamber 111. A flush port 114 (*e.g.,* second port) defining a fluid channel 115 therethrough may be disposed along (*e.g.,* attached to, integrally formed with, etc.) a middle portion of the housing 110. A fitting 116 may be disposed around a distal portion of the housing 110. In various embodiments, the housing 110 may be configured to rotate 360° (*e.g.,* move axially) within the fitting 116 to alter a position of the flush port 114 relative to a longitudinal axis of the adjustable assembly 100 and/or rotate (*e.g.,* alter an axial position of) a radial ultrasound probe extending through the housing 110 (as discussed below). An outer surface of the distal portion of the housing may include a surface feature 118 configured to frictionally engage a corresponding inner surface of the fitting 116. By way of non-limiting example, the surface feature may include a rubber seal or O-ring configured to maintain or lock an axial position of the housing 110 relative to the fitting 116 until a threshold level of rotational force is exerted on the housing 110 (*e.g.,* a sufficient amount of force exerted by a physician's hand). In various embodiments, an outer surface of the housing 110 and/or flush port 114 may include a non-slip surface (*e.g.*, over-molded or coated with rubber, etc.) to provide a physician with sufficient grip to manipulate the housing 110, *e.g.,* when wearing wet gloves, etc. An arm or projection 120 may extend from an outer surface of the fitting 116 to anchor or lock the housing of the probe assembly within a handle 140 of a catheter with radial ultrasound and needle biopsy capability (FIG. 3A).

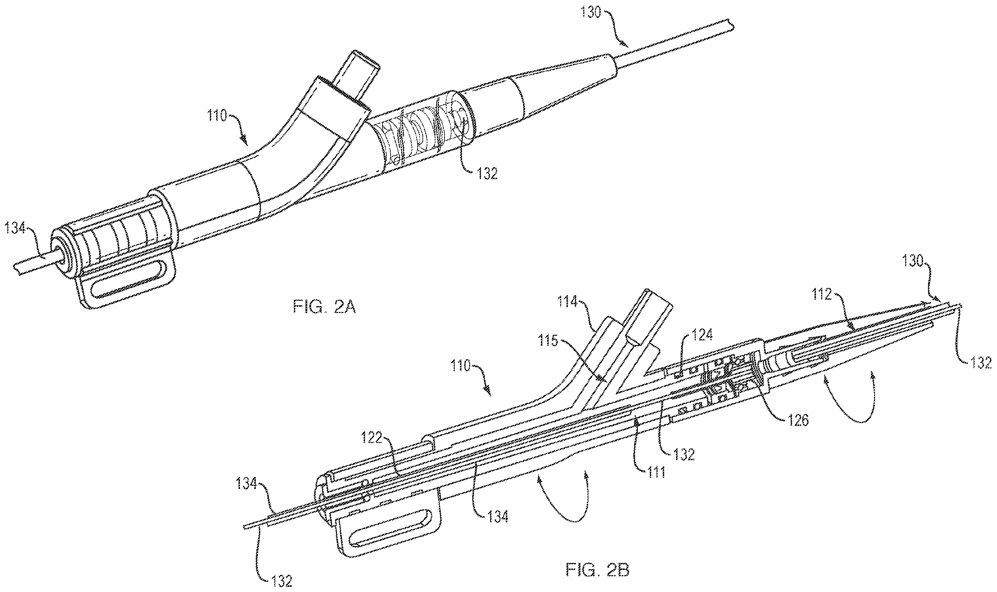

In one embodiment, a first seal 122 (*e.g.,* O-ring, etc.) may be disposed within a distal portion of the internal chamber 111 (*e.g.,* proximal to a distal opening of the housing 110) and a second seal 124 may be disposed within a proximal portion of the internal chamber 111 (*e.g.,* distal to a proximal opening of the housing 110). The first and second seals 122, 124 may be configured to prevent fluid introduced (*e.g.,* flushed) through the fluid channel 115 of the flush port 114 from exiting the internal chamber 111 (*e.g*., flowing/leaking distally beyond the first seal 122 or proximally beyond the second seal 124). A bearing 126 may be disposed within the proximal portion of the internal chamber 111 proximal to the second seal 124. In various embodiments, the housing 110 and ultrasound port 112 may be configured to receive a proximal portion of a radial ultrasound probe 130 therethrough (FIG. 2A-2B). The bearing 126 may be configured to receive an outer surface of the radial ultrasound probe 130 to support/facilitate rotation of the radial ultrasound probe 130 within the housing 110.

Referring to FIGS. 2A-2B, in one embodiment, a proximal portion of a radial ultrasound probe 130 may extend through the ultrasound port 112 and internal chamber 111 of the housing 110. In various embodiments, the radial ultrasound probe 130 may include a drive cable 132 rotatably disposed within a sheath 134. A radial ultrasound transducer (not shown) may be attached to a distal end of the drive cable 132, *e.g.,* to provide an ultrasound image within a body lumen. A proximal portion of the drive cable 132 may extend through the probe assembly 100, including the internal chamber 111, and beyond a proximal end of the housing 110 to connect to an external motor drive unit (MDU). In addition, a proximal end of the sheath 134 may extend into the internal chamber 111 such that an open proximal end of the sheath 134 is disposed between the first and second seals 122, 124. A distal portion of the radial ultrasound probe (*e.g.,* drive cable 132 and sheath 134) may extend beyond a distal end of the housing 110, *e.g.,* through a handle 140 of a catheter with radial ultrasound and needle biopsy capability (FIGS. 3A-3D) and a lumen of a dual-lumen shaft (not shown) attached to the catheter handle. The MDU may be configured to rotate the drive cable 132 at the requisite high-speed of rotation within the internal chamber 111 and throughout the full length of the sheath 134 to impart the requisite high speed of rotation to the radial ultrasound transducer. In various embodiments, the bearing 126 may be configured to dampen, insulate or otherwise minimize noise, vibrations or other outside forces acting on the housing 110 that might interfere with or corrupt the ultrasound signal propagated through the radial ultrasound probe 130 (*e.g.,* along/through drive cable 132). In addition, or alternatively, the proximal portion of the housing, *e.g.*, which includes the ultrasound port 112, may include a support structure configured to eliminate or reduce bending or kinking of the proximal portion of the radial ultrasound probe 130 (*e.g.,* sheath 134 and/or drive cable 132) and/or dampen outside forces, which may impair or otherwise negatively affect ultrasound image quality.

Referring to FIGS. 3A-3D, in one embodiment, a system 200 of the present disclosure may include an adjustable probe assembly 100 disposed within handle 140 of a catheter with radial ultrasound and needle biopsy capability. A radial ultrasound probe 130 may extend through the housing 110 such that a drive cable of the radial ultrasound probe may be connected to an external MDU and a distal portion of the radial ultrasound probe may extend through a shaft (*e.g.,* a dual-lumen shaft) of a catheter (not shown) attached to a distal end of the handle 140. In various embodiments, the dual-lumen catheter may extend through a working channel of an endoscope, *e.g*., within a body lumen of a patient. In various embodiments, the adjustable probe assembly 100 may be configured to move laterally and/or axially relative to (*e.g*., within) the handle 140. For example, the arm or projection 120 may be disposed between first and second surface features 142, 144 extending from an inner wall of the handle 140. In various embodiments, the first surface feature 142 may define an opening configured to receive a corresponding lock button/tab to lock a needle slider associated with the handle 140 in position, while surface 144 is a boundary of the handle where the probe assembly exits the handle proximally. A lateral position of the probe assembly 100 within the handle 140 may be varied by moving (*e.g*., sliding) the housing 110 distally and proximally between the first and second surface features 142, 144. In various embodiments, the lateral position of the probe assembly within the handle 140 may provide (*e.g.,* set) the desired length of the radial ultrasound probe extending through and distal to the end of a lumen of the dual-lumen catheter. For example, the lateral position of the probe assembly 100 within the handle 140 may be set or adjusted to vary the position of a radial ultrasound transducer at the distal end of the radial ultrasound probe relative to a distal end of the shaft (*e.g*., dual-lumen shaft) of the catheter within which the radial ultrasound probe is housed. In one embodiment, the ability to alter/adjust a lateral position of the probe assembly 110 within the handle 140 may provide a physician with fine-tune lateral control of the radial ultrasound probe within and extending from the dual-lumen catheter (*e.g.,* to adjust the location of the radial ultrasound probe relative to a target pulmonary nodule) without adjusting/altering the position of the catheter handle within the working channel of the endoscope, or the position of the endoscope itself through which the dual-lumen catheter of the handle 140 extends. In various embodiments, a set-screw or other locking member (not shown) may extend through a wall of the handle 140 to engage/contact an outer surface of the fitting 116 to lock/secure the housing 110 at the desired lateral position within the handle 140. With the position of the radial ultrasound transducer set (*e.g.,* relative to the distal end of the dual-lumen catheter), the handle 140, and probe assembly 100 attached thereto, may be distally advanced and/or proximally retracted to distally advance and/or proximally retract the dual-lumen catheter within/through the endoscope working channel, *e.g.,* to advance the radial ultrasound transducer into the peripheral regions of the lung using ultrasound guidance. In addition, the housing 110 may be rotated relative to the handle 140 to axially rotate the radial ultrasound probe within the dual-lumen catheter. The proximal portion of the housing (*e.g*., defining the ultrasound port 112) may also be rotated independent of the remaining portion of the housing 110 (*e.g.,* including flush port 114 and fitting 116) to rotate the radial ultrasound probe within the dual-lumen catheter. The axial rotation of the flush port 114 with the housing 110 may orient the flush port 114 to a position that is accessible to the physician so that fluid may be flushed through the channel 115 into the internal chamber 111 and through the sheath 134. In one embodiment, a proximal end of the flush port 114 may be configured to receive a fluid source 150 (FIGS. 3B-3C) configured to flush fluid through the fluid channel 115 into the internal chamber 111 and through the sheath 134. In various embodiments, the first and second seals 122, 124 may prevent fluid from exiting the housing 110 (*e.g.,* leaking) as the housing 110 is moved laterally and/or axially within the handle 140. Other adjustable ultrasound port and flush port techniques, features, and/or components that may be used herein are disclosed in U.S. Non-Provisional Patent Application titled "Devices to Access Peripheral Regions of the Lung for Direct Visualization with Tool Attachment" and/or U.S. Non-Provisional Patent Application titled "Medical Imaging Devices, Systems, and Methods'.

All of the devices disclosed and claimed herein can be made and executed without undue experimentation in light of the present disclosure. While the devices of this disclosure have been described in terms of preferred embodiments, it may be apparent to those of skill in the art that variations can be applied to the devices without departing from the scope of the disclosure. All such similar substitutes and modifications apparent to those skilled in the art are deemed to be within the scope of the disclosure as defined by the appended claims.

## Claims

1. An adjustable probe assembly (100), comprising:
a housing (110) defining an internal chamber (111), the housing configured to couple to a handle (140) of a catheter;
an ultrasound port (112) formed within a proximal portion of the housing (110), wherein the ultrasound port (112) is coextensive with the internal chamber (111) and configured to receive a radial ultrasound probe (130) therethrough;
a flush port (114) disposed along a middle portion of the housing (110), the flush port (114) defining a fluid channel (115) therethrough, wherein the fluid channel (115) is coextensive with the internal chamber (111); and
a fitting (116) disposed around a distal portion of the housing (110);
wherein the housing (110) is configured to rotate relative to the fitting (116) to alter a position of the flush port (114) around the longitudinal axis of the probe assembly relative to the fitting (116); and
wherein the adjustable probe assembly (100) is configured to move along the longitudinal axis of the probe assembly relative to the handle (140) of the catheter.

2. The adjustable probe assembly (100) of claim 1, wherein an outer surface of the distal portion of the housing (110) includes a surface feature (118) configured to frictionally engage an inner surface of the fitting (116).

3. The adjustable probe assembly (100) of any of claims 1-2, wherein the fitting (116) includes a projection (120).

4. The adjustable probe assembly (100) of any of claims 1-3, further comprising a first seal (122) disposed within a distal portion of the internal chamber (111) and a second seal (124) disposed within a proximal portion of the internal chamber (111).

5. The adjustable probe assembly (100) of claim 4, further comprising a bearing (126) disposed within a proximal portion of the internal chamber (111) and proximal to the second seal (124).

6. The adjustable probe assembly (100) of claim 5, wherein the housing (110) is configured to receive the radial ultrasound probe therethrough.

7. The adjustable probe assembly (100) of claim 6, wherein the bearing (126) is configured to support rotation of the radial ultrasound probe (130).

8. The adjustable probe assembly (100) of claim 7, wherein the radial ultrasound probe (130) includes a drive cable (132), and wherein the drive cable (132) extends through the ultrasound port (112) and is supported by the bearing (126).

9. The adjustable probe assembly (100) of claim 8, wherein the radial ultrasound probe (130) includes a sheath (134), and wherein a proximal end of the sheath (134) is disposed within the housing (110) between the first and second seals (122, 124).

10. The adjustable assembly (100) of claim 9, wherein the housing (110) is configured to rotate relative to the fitting (116) to alter an axial position of the radial ultrasound probe (130), and wherein the proximal portion of the housing (110) is configured to rotate relative to a remaining portion of the housing (110) to alter an axial position of the radial ultrasound probe (130).

11. The adjustable assembly (100) of any one of the preceding claims, wherein the housing (110) is configured to rotate relative to the fitting (116) to alter an axial position of the flush port (114) around the longitudinal axis of the probe assembly relative to the fitting (116); and
wherein movement of the adjustable probe assembly (100) along the longitudinal axis of the probe assembly relative to the handle (140) of the catheter is a lateral movement of the adjustable probe assembly (100) relative to the handle (140) of the catheter.

12. A system (200), comprising:
a handle (140) of a catheter;
the adjustable probe assembly (100) according to any of the preceding claims attached to the handle (140), wherein the adjustable probe assembly (100) is configured to move around the longitudinal axis of the probe assembly relative to the handle (140); and
a radial ultrasound probe (130) extending through the adjustable probe assembly (100), the handle (140) and a shaft of the catheter.

13. The system (200) of claim 12, wherein the flush port (114) includes a fluid channel (115) configured to deliver fluid into the internal chamber (111) and through a/the sheath (134) of the radial ultrasound probe (130).

14. The system (200) of any of claims 12-13 as depending from claim 4, wherein the first and second seals (122, 124) prevent fluid introduced through the flush port (114) from exiting the internal chamber (111).

15. The system (200) of any of claims 12-14, wherein movement of the adjustable probe assembly (100) around the longitudinal axis of the probe assembly relative to the handle (140) is an axial movement of the adjustable probe assembly (100) relative to the handle (140).

## Patentansprüche

1. Einstellbare Sondenanordnung (100), die aufweist:
ein Gehäuse (110), das eine Innenkammer (111) definiert, wobei das Gehäuse dazu konfiguriert ist, mit einem Griff (140) eines Katheters zu koppeln,
einen Ultraschallport (112), der in einem proximalen Abschnitt des Gehäuses (110) gebildet ist, wobei sich der Ultraschallport (112) mit der Innenkammer (111) ko-erstreckt und dazu konfiguriert ist, eine Radialultraschallsonde (130) dort hindurch aufzunehmen;
einen Spülport (114), der entlang eines Mittelabschnitts des Gehäuses (110) angeordnet ist, wobei der Spülport (114) einen Fluidkanal (115) dort hindurch definiert, wobei sich der Fluidkanal (115) mit der Innenkammer (111) ko-erstreckt; und
ein Anschlussstück (116), das um einen distalen Abschnitt des Gehäuses (110) angeordnet ist;
wobei das Gehäuse (110) dazu konfiguriert ist, sich relativ zum Anschlussstück (116) zu drehen, um eine Position des Spülports (114) um die Längsachse der Sondenanordnung relativ zum Anschlussstück (116) zu ändern; und
wobei die einstellbare Sondenanordnung (100) dazu konfiguriert ist, sich entlang der Längsachse der Sondenanordnung relativ zum Griff (140) des Katheters zu bewegen.

2. Einstellbare Sondenanordnung (100) nach Anspruch 1, wobei eine Außenfläche des distalen Abschnitts des Gehäuses (110) ein Oberflächenmerkmal (118) aufweist, das dazu konfiguriert ist, einen reibschlüssigen Eingriff mit einer Innenfläche des Anschlussstücks (116) herzustellen.

3. Einstellbare Sondenanordnung (100) nach einem der Ansprüche 1 bis 2, wobei das Anschlussstück (116) einen Vorsprung (120) aufweist.

4. Einstellbare Sondenanordnung (100) nach einem der Ansprüche 1 bis 3, ferner aufweisend eine erste Dichtung (122), die in einem distalen Abschnitt der Innenkammer (111) angeordnet ist, und eine zweite Dichtung (124), die in einem proximalen Abschnitt der Innenkammer (111) angeordnet ist.

5. Einstellbare Sondenanordnung (100) nach Anspruch 4, ferner aufweisend ein Lager (126), das in einem proximalen Abschnitt der Innenkammer (111) und proximal zur zweiten Dichtung (124) angeordnet ist.

6. Einstellbare Sondenanordnung (100) nach Anspruch 5, wobei das Gehäuse (110) dazu konfiguriert ist, die Radialultraschallsonde dort hindurch aufzunehmen.

7. Einstellbare Sondenanordnung (100) nach Anspruch 6, wobei das Lager (126) dazu konfiguriert ist, Drehung der Radialultraschallsonde (130) zu unterstützen.

8. Einstellbare Sondenanordnung (100) nach Anspruch 7, wobei die Radialultraschallsonde (130) ein Betriebskabel (132) aufweist und wobei sich das Betriebskabel (132) durch den Ultraschallport (112) erstreckt und vom Lager (126) gestützt wird.

9. Einstellbare Sondenanordnung (100) nach Anspruch 8, wobei die Radialultraschallsonde (130) eine Hülle (134) aufweist und wobei ein proximales Ende der Hülle (134) im Gehäuse (110) zwischen der ersten und zweiten Dichtung (122, 124) angeordnet ist.

10. Einstellbare Anordnung (100) nach Anspruch 9, wobei das Gehäuse (110) dazu konfiguriert ist, sich relativ zum Anschlussstück (116) zu drehen, um eine Axialposition der Radialultraschallsonde (130) zu ändern, und wobei der proximale Abschnitt des Gehäuses (110) dazu konfiguriert ist, sich relativ zu einem übrigen Abschnitt des Gehäuses (110) zu drehen, um eine Axialposition der Radialultraschallsonde (130) zu ändern.

11. Einstellbare Anordnung (100) nach einem der vorstehenden Ansprüche, wobei das Gehäuse (110) dazu konfiguriert ist, sich relativ zum Anschlussstück (116) zu drehen, um eine Axialposition des Spülports (114) um die Längsachse der Sondenanordnung relativ zum Anschlussstück (116) zu ändern; und
wobei Bewegung der einstellbaren Sondenanordnung (100) entlang der Längsachse der Sondenanordnung relativ zum Griff (140) des Katheters eine Lateralbewegung der einstellbaren Sondenanordnung (100) relativ zum Griff (140) des Katheters ist.

12. System (200), das aufweist:
einen Griff (140) eines Katheters;
die einstellbare Sondenanordnung (100) nach einem der vorstehenden Ansprüche, die am Griff (140) angebracht ist, wobei die einstellbare Sondenanordnung (100) dazu konfiguriert ist, sich um die Längsachse der Sondenanordnung relativ zum Griff (140) zu bewegen; und
eine Radialultraschallsonde (130), die sich durch die einstellbare Sondenanordnung (100), den Griff (140) und einen Schaft des Katheters erstreckt.

13. System (200) nach Anspruch 12, wobei der Spülport (114) einen Fluidkanal (115) aufweist, der dazu konfiguriert ist, Fluid in die Innenkammer (111) und durch eine/die Hülle (134) der Radialultraschallsonde (130) zuzuführen.

14. System (200) nach einem der Ansprüche 12 bis 13, wenn abhängig von Anspruch 4, wobei die erste und zweite Dichtung (122, 124) über den Spülport (114) eingeleitetes Fluid daran hindern, aus der Innenkammer (111) auszutreten.

15. System (200) nach einem der Ansprüche 12 bis 14, wobei Bewegung der einstellbaren Sondenanordnung (100) um die Längsachse der Sondenanordnung relativ zum Griff (140) eine Axialbewegung der einstellbaren Sondenanordnung (100) relativ zum Griff (140) ist.

## Revendications

1. Ensemble de sonde réglable (100) comprenant :
un boîtier (110) définissant une chambre interne (111), le boîtier étant configuré pour se coupler à une poignée (140) d'un cathéter ;
un orifice ultrasonore (112) formé dans une partie proximale du boîtier (110), dans lequel l'orifice ultrasonore (112) est coextensif avec la chambre interne (111) et configuré pour recevoir une sonde radiale ultrasonore (130) à travers ce dernier ;
un orifice de rinçage (114) disposé le long d'une partie centrale du boîtier (110), l'orifice de rinçage (114) définissant un canal de fluide (115) à travers ce dernier, dans lequel le canal de fluide (115) est coextensif avec la chambre interne (111) ; et
un raccord (116) disposé autour d'une partie distale du boîtier (110) ;
dans lequel le boîtier (110) est configuré pour tourner par rapport au raccord (116) afin de modifier une position de l'orifice de rinçage (114) autour de l'axe longitudinal de l'ensemble de sonde par rapport au raccord (116) ; et
dans lequel l'ensemble de sonde réglable (100) est configuré pour se déplacer le long de l'axe longitudinal de l'ensemble de sonde par rapport à la poignée (140) du cathéter.

2. Ensemble de sonde réglable (100) selon la revendication 1, dans lequel une surface externe de la partie distale du boîtier (110) comprend une caractéristique de surface (118) configurée pour mettre en prise, par friction, une surface interne du raccord (116).

3. Ensemble de sonde réglable (100) selon l'une quelconque des revendications 1 à 2, dans lequel le raccord (116) comprend une saillie (120).

4. Ensemble de sonde réglable (100) selon l'une quelconque des revendications 1 à 3, comprenant en outre un premier joint d'étanchéité (122) disposé à l'intérieur d'une partie distale de la chambre interne (111) et un deuxième joint d'étanchéité (124) disposé à l'intérieur d'une partie proximale de la chambre interne (111).

5. Ensemble de sonde réglable (100) selon la revendication 4, comprenant en outre un palier (126) disposé à l'intérieur d'une partie proximale de la chambre interne (111) et proximal par rapport au deuxième joint d'étanchéité (124).

6. Ensemble de sonde réglable (100) selon la revendication 5, dans lequel le boîtier (110) est configuré pour recevoir la sonde radiale ultrasonore à travers ce dernier.

7. Ensemble de sonde réglable (100) selon la revendication 6, dans lequel le palier (126) est configuré pour supporter la rotation de la sonde radiale ultrasonore (130).

8. Ensemble de sonde réglable (100) selon la revendication 7, dans lequel la sonde radiale ultrasonore (130) comprend un câble d'entraînement (132) et dans lequel le câble d'entraînement (132) s'étend à travers l'orifice ultrasonore (112) et est supporté par le palier (126).

9. Ensemble de sonde réglable (100) selon la revendication 8, dans lequel la sonde radiale ultrasonore (130) comprend une gaine (134), et dans lequel une extrémité proximale de la gaine (134) est disposée à l'intérieur du boîtier (110) entre les premier et deuxième joints d'étanchéité (122, 124).

10. Ensemble réglable (100) selon la revendication 9, dans lequel le boîtier (110) est configuré pour tourner par rapport au raccord (116) afin de modifier une position axiale de la sonde radiale ultrasonore (130), et dans lequel la partie proximale du boîtier (110) est configurée pour tourner par rapport à une partie résiduelle du boîtier (110) afin de modifier une position axiale de la sonde radiale ultrasonore (130).

11. Ensemble réglable (100) selon l'une quelconque des revendications précédentes, dans lequel le boîtier (110) est configuré pour tourner par rapport au raccord (116) afin de modifier une position axiale de l'orifice de rinçage (114) autour de l'axe longitudinal de l'ensemble de sonde par rapport au raccord (116) ; et
dans lequel le déplacement de l'ensemble de sonde réglable (100) le long de l'axe longitudinal de l'ensemble de sonde par rapport à la poignée (140) du cathéter est un déplacement latéral de l'ensemble de sonde réglable (100) par rapport à la poignée (140) du cathéter.

12. Système (200) comprenant :
une poignée (140) d'un cathéter ;
l'ensemble de sonde réglable (100) selon l'une quelconque des revendications précédentes, fixé à la poignée (140), dans lequel l'ensemble de sonde réglable (100) est configuré pour se déplacer autour de l'axe longitudinal de l'ensemble de sonde par rapport à la poignée (140) ; et
une sonde radiale ultrasonore (130) s'étendant à travers l'ensemble de sonde réglable (100), la poignée (140) et une tige du cathéter.

13. Système (200) selon la revendication 12, dans lequel l'orifice de rinçage (114) comprend un canal de fluide (115) configuré pour distribuer le fluide dans la chambre interne (111) et à travers une/la gaine (134) de la sonde radiale ultrasonore (130).

14. Système (200) selon l'une quelconque des revendications 12 à 13, lorsqu'elle dépend de la revendication 4, dans lequel les premier et deuxième joints d'étanchéité (122, 124) empêchent que le fluide introduit par l'orifice de rinçage (114) de sortir de la chambre interne (111).

15. Système (200) selon l'une quelconque des revendications 12 à 14, dans lequel le déplacement de l'ensemble de sonde réglable (100) autour de l'axe longitudinal de l'ensemble de sonde par rapport à la poignée (140) est un déplacement axial de l'ensemble de sonde réglable (100) par rapport à la poignée (140).
